# EUROPEAN PATENT APPLICATION

(11) **EP 2 084 958 A1**
(43) Date of publication of application: **05.08.2009**
(21) Application number: 08001894.8
(22) Date of filing: 01.02.2008
(51) Int. Cl.: A01H 1/00, A01H 5/00

(54) **Seedless eggplant**

(71) Applicant: Rijk Zwaan Zaadteelt en Zaadhandel B.V., 2678 KX De Lier (NL)
(72) Inventor: The designation of the inventor has not yet been filed
(74) Representative: Van Someren, Petronella F. H. M.

(57) **Abstract**

The invention relates to a method for producing a seedless eggplant comprising the steps of providing a first tetraploid eggplant parent plant; providing a second eggplant parent plant that is diploid; crossing the first and the second parent plant to obtain a population of progeny plants; and selecting the triploid plants from the population of progeny plants as the seedless eggplant.

## Description

The present invention relates to a seedless eggplant and to a method for producing such plant.

The eggplant or aubergine (*Solanum melongena*) is a solanaceous plant bearing a fruit of the same name, commonly used as a vegetable in cooking. It is closely related to the tomato and potato. Because of its large, pendulous, purple or white fruit, the eggplant is an important food crop. It has been cultivated in southern and eastern Asian countries since prehistory but is now also grown in the Western world. The fruit contains numerous small, soft seeds. The presence of seeds in ready-to-harvest fruits is considered as a neagtive quality. This is *inter alia* because seeds can lead to undesired browning of the flesh.

From a commercial point of view seedlessness is a very desirable trait in edible fruit and vegetables, like the eggplant. In some species, seedlessness is the result of parthenocarpy, where fruits set without fertilization. In certain species parthenocarpic fruit set requires pollination or another stimulus, in particular spraying the flowers with plant growth regulators, such as gibberellin, auxin and cytokinin. This is termed artificial parthenocarpy.

Parthenocarpic eggplant varieties are known (e.g. Talina, Galine). However, during winter cultivation of such eggplant varieties fruit production may be hampered by suboptimal environmental conditions. These are usually counteracted by treating flower buds with plant growth regulators. However, phytohormonal treatments make the production process more expensive due to the cost of both chemicals and labour. In addition, the use of hormones is under discussion or prohibited in some countries.

Parthenocarpy has also been genetically engineered in eggplant by using the *DefH9-iaaM* gene. The *iaaM* gene codes for tryptophan monoxygenase and confers auxin synthesis, while the *DefH9* controlling regions drive expression of the gene specifically in the ovules and placenta. This led to significant increase in fruit production concomitant with a reduction in cultivation costs. However, in many countries genetically modified crops are not well accepted.

It is therefore the object of the present invention to provide a seedless eggplant that does not have the above stated drawbacks.

This is achieved by the present invention by means of a method for producing a seedless eggplant comprising the steps of:
a) providing a first tetraploid eggplant parent plant;
b) providing a second eggplant parent plant that is diploid;
c) crossing the first and the second parent plant to obtain a population of progeny plants; and
d) selecting the triploid plants from the population of progeny plants as the seedless eggplant.

Tetraploid parent plants are suitably produced by doubling the chromosomes of a diploid seedling or explant.

In a preferred embodiment the chromosomes are doubled by treatment with colchicine.

A suitable method for colchicine doubling consists of growing seedlings from eggplant seeds and preparing diploid cuttings therefrom. The cuttings are then rooted, preferably on an artificial medium. After roots have developed the top and almost all, preferably all except one, of the others leaves are removed from the cuttings. The remaining leaf of the explant is contacted with a solution of colchicine for a certain amount of time to effect doubling of the chromosomes. After treatment the cuttings are grown until shoots develop. These shoots are tested for tetraploidy. Diploid shoots are removed.

Tetraploid plants that are thus produced can be used as a parent plant in the production of triploid eggplants. Preferably, the tetraploid parent is the mother plant and the diploid parent is the father plant.

In a preferred embodiment of the invention the tetraploid parent plant is a plant grown from AB5123-tetra seeds deposited on 13 November 2007 with the NCIMB in Aberdeen under deposit accession number NCIMB 41516.

The invention further relates to triploid seedless eggplants obtainable by performing the method described above. In a preferred embodiments such seedless eggplants are obtainable by:
a) growing a first parent plant from AB5123-tetra seeds;
b) crossing the first parent plant with a second diploid eggplant parent plant to obtain a population of progeny plants;
c) selecting the triploid plants from the population of progeny plants as the seedless eggplant.

The eggplants of the present invention produce fruits without a pollination or treatment with plant growth regulators.

"Seedless" as used in the present application means the complete or almost complete absence of viable seeds. The fruits may still contain remnants of seeds, such as pellicles or skins.

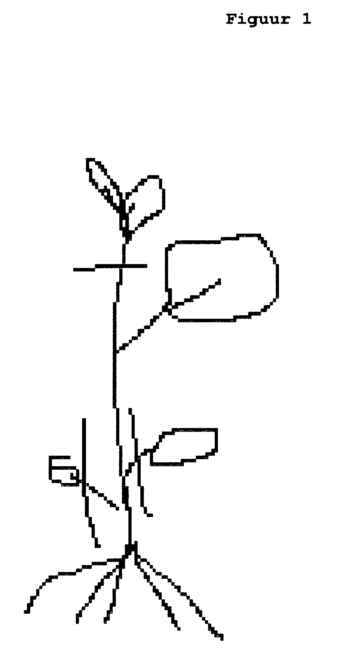

The present invention will be further illustrated in the examples that follow and that are not intended to limit the invention in any way. In the examples reference is made to the figure which shows preparation of explant for chromosome doubling.

### EXAMPLES

### EXAMPLE 1

### Production of tetraploid parent eggplant

### 1. Chromosome doubling by means of colchicine

Seeds of a diploid eggplant were sterilized in 0.5% chlorine during 30 min and subsequently rinsed with demineralized water until all foam was removed. Seeds (10 seeds on each plate) were sown on ½MS10 medium. The seeds were germinated at 25°C under 16 hrs of light. After 2 weeks cuttings were prepared by taking the hypocotyls from the seedlings. These were planted in MS20 medium.

After three weeks the cuttings were treated with colchicine. The top and all except one leaf of the cuttings were removed according to the **Figure** to obtain explants. A colchicine solution was prepared by dissolving 5 g colchicone (Sigma C-9754) in 1000 ml water and filtersterilizing. The explants were placed upside down in the solution for 2 hours. The roots did not touch the colchicine solution.

After 2 hours the explants were rinsed with sterile demineralized water and put on MS20 medium. After they started growing the plants were potted in soil. Shoots were tested for their ploidy by means of the method as described in De Laat et al., Plant Breeding 99(4), 303-307 (1987).

Diploid shoots were removed.

### 1. Results

The results of various experiments with different parent plants is shown in the Table.

**Table**

| **parental line** | **number of potted plants** | **number of tetraploid plants** | **% of tetrapoid plants** |
|---|---|---|---|
| T18 | 67 | 40 | 60 |
| T44 | 68 | 37 | 54 |
| AB5020 | 30 | 25 | 83 |
| AB5121 | 26 | 13 | 50 |

### EXAMPLE 2

### Production of triploid eggplant seeds

The seeds obtained from Example 1 were sown under the conditions used by commercial plant raisers. Ploidy level of the plants obtained was checked by DNA analysis.

Tetraploid plants obtained in this way were used as a mother line for seed production and crossed with a diploid father line in order to reconstruct the original diploid hybrid as a triploid. Seeds obtained from this cross were harvested and plants obtained were checked for ploidy level by flow-cytometry. All seeds were found to be triploid.

### EXAMPLE 3

### Analysis of triploid eggplant hybrid

Triploid hybrid seeds obtained from Example 2 were sown under the conditions used by commercial plant raisers and planted in a hybrid testing field. The ploidy level of the hybrid plants obtained was checked by DNA analysis. All hybrid plants were found to be triploid. Fruit set and fruit quality of the triploid hybrid plants were analyzed and compared with the original diploid hybrid plants.

## Claims

1. Method for producing a seedless eggplant comprising the steps of:
a) providing a first tetraploid eggplant parent plant;
b) providing a second eggplant parent plant that is diploid;
c) crossing the first and the second parent plant to obtain a population of progeny plants; and
d) selecting the triploid plants from the population of progeny plants as the seedless eggplant.

2. Method as claimed in claim 1, wherein the tetraploid eggplant parent plant is produced by doubling the chromosomes of a diploid seedling or explant.

3. Method as claimed in claim 2, wherein the chromosomes are doubled by treatment with colchicine.

4. Method as claimed in any one of the claims 1-3, wherein the tetraploid parent is the mother plant and the diploid parent is the father plant.

5. Method as claimed in claim 1, wherein the tetraploid parent plant is a plant grown from AB5123-tetra seeds (NCIMB41516).

6. Seedless eggplant obtainable by performing the method as claimed in any one of the claims 1-5.

7. Seedless eggplant obtainable by:
a) growing a first parent plant from AB5123-tetra seeds;
b) crossing the first parent plant with a second diploid eggplant parent plant to obtain a population of progeny plants;
c) selecting the triploid plants from the population of progeny plants as the seedless eggplant.

8. Seedless eggplant as claimed in claim 6 or 7, which eggplant is a hybrid.
